(19)

Europäisches
Patentamt
European
Patent Office
Office européen
des brevets

(11) **EP 4 385 618 A1**

(12) **EUROPEAN PATENT APPLICATION**

(43) Date of publication:
**19.06.2024 Bulletin 2024/25**

(21) Application number: **22306901.4**

(22) Date of filing: **16.12.2022**

(51) International Patent Classification (IPC):
**B01J 20/26** (2006.01)    **A61K 48/00** (2006.01)
**B01D 15/08** (2006.01)    **B01J 20/32** (2006.01)

(52) Cooperative Patent Classification (CPC):
**B01J 20/3204; B01D 15/08; B01J 20/262;
B01J 20/3272; B01J 20/3282; B01J 20/3285;
B01J 20/3289; B01J 20/3293; C12N 7/00;
C12N 15/86;** C12N 2750/14151

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Chiral Technologies Europe SAS
67404 Illkirch Cedex (FR)**

(72) Inventors:
• **Crestey, Loic
67404 Illkirch Cedex (FR)**
• **Morishita, Yasuto
67404 Illkirch Cedex (FR)**
• **Franco, Pilar
67404 Illkirch Cedex (FR)**

(74) Representative: **Grünecker Patent- und
Rechtsanwälte
PartG mbB
Leopoldstraße 4
80802 München (DE)**

(54) **COMPOSITE MATERIAL FOR BIOSEPARATIONS**

(57)    The present invention relates to composite materials useful for purifying large biomolecules, such as proteins, viruses, virus-like particles and extracellular vesicles obtained from biological feedstocks. The composite materials of the invention comprise a porous support being filled with a first polymer which is a cross-linked cationic polymer, and a second polymer which is an anionic polymer and is covalently bonded to the external surface of the cross-linked polymer-filled porous support.

EP 4 385 618 A1

**Description**

<u>**TECHNICAL FIELD**</u>

The present invention relates to composite materials useful for purifying large biomolecules from biological feedstocks in high yields.

<u>**BACKGROUND OF THE INVENTION**</u>

The relevance of proteins, viruses, virus-like particles and extracellular vesicles for use as biopharmaceuticals has continuously increased during the last decades in many therapeutic and diagnostic applications. The number of approved therapeutic vaccine and virus vectors thereof for Cell and Gene therapies increases each year.

Gene delivery is a promising method for the treatment of acquired and inherited diseases. A number of viral-based systems for gene transfer purposes have been described, including adeno-associated virus (AAV)-based systems.

Extracellular vesicles (EVs) are nano-sized vesicles (generally less than 1000 nm in hydrodynamic diameter) that are released by EV-producing cells into the extracellular environment. EVs and in particular exosomes have been shown to be able to transport protein biologics, such as antibodies and decoy receptors, into target cells, enabling an entirely novel form of advanced biological therapeutics harnessing the properties of EVs in combination with the specificity of recombinant proteins.

At research level, a target virus is purified from the virus culture medium using ultracentrifugation, affinity packing, etc. In the manufacturing process, multiple chromatographies (ion exchange, affinity, etc.) are combined for purification. The principle of chromatography is mainly based on a method called capture mode, in which a packing material captures a target substance. A method called flow-through mode (hereinafter FT) that captures only impurities and does not capture the target virus has begun to be considered as a packing material. Examples include Capto Core, which has size exclusion and anion exchange functions, and MabXpure (WO 2019/170634).

However, Capto Core, a commercially available FT packing material, has poor removal efficiency of some impurities (such as DNA) due to the absence of functional groups on the surface shell, and the purification recovery rate is low depending on the target molecular size.

Furthermore, the FT packing material of WO 2019/170634 adsorbs both viruses and impurities and cannot be purified.

US 2016/367966 discloses a separation matrix for purification of biological particles, comprising a plurality of particles having a porous core entity and a porous shell entity covering the core entity, wherein the core entity comprises at least 50 micromole/ml primary amines present on covalently attached ligands displaying at least two primary amines per ligand and the shell entity comprises less than 20 micromole/ml primary amines

US 2019/111419 describes a high resolution separation matrix for purification of biomacromolecules such as proteins comprising a plurality of particles with a core region comprising a grafted polymer and a shell region, wherein the shell region is accessible to a target biomacromolecule and the core region is less accessible to the target biomacromolecule than the shell region.

WO 2019/206940 relates to a method for adenovirus purification comprising the steps of capturing adenovirus from an adenovirus-containing cell culture harvest on an anion exchanger resin, eluting said adenovirus with a shallow conductivity gradient with an increasing salt concentration, adding said eluted adenovirus to a shell bead resin comprising a porous shell and a porous core, wherein the core is provided with hydrophobic interaction ligands and the shell is not provided with any ligands, and eluting said adenovirus from said shell bead resin in the flow through.

WO 2019/006390 discloses purification and production methods for recombinant adeno-associated viral (rAAV) vector particles. However, the methods include at least two column chromatography steps.

US 9,782,468 describes a method of purification of polysaccharide protein conjugates using mixed mode chromatography comprising contacting the polysaccharide protein conjugate with a mixed mode resin, and collecting unbound purified polysaccharide protein conjugate in the flowthrough. The mixed mode resin comprises of an inert shell and an activated core.

K. Reiter et al., Separation of virus-like particles and extracellular vesicles by flow-through and heparin affinity chromatography, Journal of Chromatography A, Volume 1588, Pages 77-84 studies the separation of enveloped virus-like particles from other extracellular vesicles in a two-step chromatographic purification method. In the first step, virus-like particles and extracellular vesicles were collected and separated from smaller impurities in a flow-through mode. The collected flow-through was further purified using heparin affinity chromatography. However, the method requires two chromatography steps.

WO 2021/092193 discloses a method of purifying an extracellular vesicle (EV) from a sample. The method comprises the steps of contacting the sample to a chromatography resin or medium under a plurality of chromatography operational parameters, collecting a fraction comprising the EV, and determining an EV yield, impurity recovery, and/or EV ligand density. Further provided is a method of identifying one or more chromatography operational parameters (e.g., binding parameters) for a chromatography for purifying an extracellular vesicle (EV) from a sample comprising the EV and an impurity. However, a plurality of chromatography operational parameters is required for the purification.

US 2021/188903 relates to the isolation and purification of EVs by utilizing chromatography matrices comprising Fc domains, to which EVs engineered to comprise Fc binding polypeptides are being attached. The document describes processes for isolating and/or purifying EVs comprising the steps of contacting a medium comprising the EVs with a chromatography matrix comprising Fc domains, allowing the EVs to adsorb to the Fc domains, and eluting the EVs by passing across the chromatography matrix a medium that releases the EVs from the Fc domains. However, the method described therein is highly specific.

Another separating method is ultracentrifugation. However, ultracentrifugation has problems such as difficulty in scaling up and the use of harmful solvents. Affinity is expensive and can only be used for specific purpose substances.

Capture mode packing adsorbs the target substance, rinses away the impurities, and then requires multiple operations to recover the target substance from the packing and a variety of solvents.

WO 95/025574 discloses a method for removing contaminants from a biological fluid comprising bringing said biological fluid into contact with a cross-linked hydrophobic polymeric network overlaying, but not covalently bound to, a porous mineral oxide matrix, having its interior porous volume substantially filled by said hydrophobic network, whereby hydrophobic and amphiphilic molecules with an average molecular mass below 10,000 Da are removed.

US 6,783,962 relates to a particulate material useful for the isolation/purification of biomacromolecules. The particulate material has a density of at least 2.5 g/ml, the particles of the particulate material have an average diameter of 5-75 $\mu$m, and the particles of the particulate material are essentially constructed of a polymeric base matrix and a non-porous core material, said core material having a density of at least 3.0 g/ml. The polymeric base matrix includes pendant groups which are positively charged at pH 4.0 or which are affinity ligands for a biomolecule.

WO 2004/073843 discloses a composite material that comprises a support member that has a plurality of pores and a macroporous cross-linked gel filling the pores of the support member. Also disclosed is a process for adsorbing a biological molecule or a biological ion from a liquid, which comprises passing a liquid containing the biological molecule or biological ion through a composite material which bears binding sites that display specific interactions for the biomolecule on the macroporous gel.

EP-A-2 545 989 discloses a composite material for chromatographic applications which comprises a porous support and a cross-linked polymer on the surface of the porous support, wherein the ratio between the pore size [nm] of the porous support and the cross-linking degree [%] of the cross-linked polymer is from 0.25 to 20 [nm/%], and wherein the cross-linking degree is from 5 to 20% based on the total number of cross-linkable groups in the cross-linked polymer.

WO 2018/050849 discloses the preparation of a composite material comprising porous silica gel with a pore size of 25 nm and a cross-linked poly(vinylformamide-co-polyvinylamine).

WO 2006/015495 relates to a composite material comprising a porous support membrane durably filled or coated with a non-cross-linked polymer. The pore size of the porous support can be within the range of 0.1 to 5 $\mu$m. The composite material can be used for protein adsorption. This reference does not mention or suggest covalently bonding a non-cross-linked polymer to the external surface of the polymer-filled porous support.

WO 2005/120701 discloses a composite material comprising: a support membrane that has a plurality of pores extending therethrough, and a macroporous crosslinked gel that is located in, and fills, the pores of the support member, in which crosslinked gel is entrapped a stabilizing polymer that is substantially water-insoluble but water swellable.

EP-A-2 027 921 describes a porous sorptive media comprising a substrate having a first external side and a second external side, both sides being porous, and a porous thickness between them, said substrate having a sorptive material substantially covering the solid matrix of the substrate and said first and second external surfaces, said sorptive material comprising a crosslinked polymer having attached primary amine groups.

WO 2011/140406 discloses a porous sorptive media comprising mixed cellulose esters supported on a non-woven substrate and coated with a crosslinked polymer having attached primary amine groups.

The present invention has been designed to overcome the limitations of existing technologies in the purification of biomolecules.

## SUMMARY OF THE INVENTION

The object of the present invention is to provide composite materials which achieve improved purification of large biomolecules, such as virus and extracellular vesicles, virus-like particle, cell or phage from biological feedstocks. The composites allow to deplete impurities such as proteins and DNA, endotoxins from feedstock with high recovery of target biomolecules.

The object of the present invention is achieved by a composite material according to appended claim 1.

Specifically, the present invention provides a composite material comprising a porous support being filled with a first cationic polymer which is cross-linked, and a second anionic polymer which is covalently bonded to the external surface of the cross-linked polymer-filled porous support.

The present invention is based on the surprising finding that in the composite material of the present invention the purification capability of the composite material is significantly improved compared to known composite materials. Adsorption and removal of contaminants (e.g., DNA, proteins derived from host cells) is achieved simply by contacting the packing material with a solution containing the target biological material and contaminants. It is now possible to purify the target biological material with high recovery. Contaminants, especially DNA, are removed better than surface shell structures such as commercially available Capto Core.

The present invention provides a composite material for purification of large biomolecules from undesired compounds contained in the same solution or suspension. The composites are particularly suited for the efficient removal of impurities from manufactured biotherapeutics, and could easily be integrated in clarification or downstream purification processes (DSP).

The composite material of the present invention can preferably separate the viral particle, virus-like particle, extracellular vesicles, cells and phages of interest from contaminants, such as HcDNA, HCP, additive proteins, in cell culture medium.

The invention is also directed to a method for producing the composite material comprising the steps of:

a) soaking a porous support with a solution or a dispersion containing a first cationic polymer, a cross-linker, and a solvent;

b) cross-linking the first cationic polymer with the cross-linker at a temperature below 250°C, and

c) covalently bonding a second anionic polymer to the external surface of the composite material obtained in step b).

Also, provided is the use of the composite material of the invention for purifying a target biological material in a feedstock.

Further, the invention provides a method for purifying a target biological material in a feedstock, said method comprising the steps of:

i) contacting the feedstock with a composite material of the invention for a sufficient time;

ii) separating the composite material from the purified feedstock;

iii) optionally, isolating the purified biological material from the feedstock; and

iv) optionally, washing the composite material with a solvent and collecting the obtained solution for further processing.

## DETAILED DESCRIPTION

COMPOSITE MATERIAL

In the present specification, the terms "composite", "composite material" and "adsorbent" are used interchangeably.

In the present specification, any reference to a "pore size" means "average pore size".

In a preferred embodiment, in combination with any of the above or below embodiments, the porous support material has an average pore size of 5 nm to 500 nm, more preferably 15 nm to 300 nm, further preferably 20 nm to 200 nm, even more preferably 25 nm to 250 nm, most preferably 30 nm to 200 nm, and particularly preferably 40 nm to 100 nm such as 50 nm to 100 nm. In the present invention, the average pore size of the porous support material is determined by mercury intrusion according to DIN 66133.

The porous support material can be a membrane, a hollow-fiber, a non-woven tissue, a monolithic or a particulate material. Particulate and monolithic porous materials are preferred. In a preferred embodiment in combination with any of the above or below embodiments, the porous support material is a particulate porous support material which has irregular or spherical shape.

In a further preferred embodiment, in combination with any of the above or below embodiments, the porous support material is composed of a metal oxide, a semi-metal oxide, a ceramic material, a zeolite, or a natural or synthetic polymeric material.

In a further preferred embodiment, in combination with any of the above or below embodiments, the porous support material is porous silica, alumina or titania particles.

In a further preferred embodiment, in combination with any of the above or below embodiments, the porous support material is porous silica gel.

In a further preferred embodiment, in combination with any of the above or below embodiments, the porous support material is a porous polysaccharide, such as cellulose, chitosan or agarose.

In a further preferred embodiment, in combination with any of the above or below embodiments, the porous support material is a porous synthetic polymer, such as polyacrylate, polymethacrylate, polyetherketone, polyalkymether, polyarylether, polyvinylalcohol, or polystyrene, or mixtures or copolymers thereof.

In a further preferred embodiment, in combination with any of the above or below embodiments, the porous support material is a particulate material with an average particle size (diameter) of 1 $\mu$m and 500 $\mu$m, preferably between 10 $\mu$m and 200 $\mu$m, more preferably 20 to 150 $\mu$m and most preferably 30 to 100 $\mu$m.

In the present specification, the average particle size (diameter) and the particle size distribution of the porous support is determined by Malvern Laser Diffraction.

In the present specification, unless otherwise specified, the term "first cationic polymer" refers to the polymer before being cross-linked.

In a preferred embodiment, in combination with any of the above or below embodiments, the first cationic polymer contains amino groups, and preferably is a polyamine. In a further preferred embodiment, in combination with any of the above or below embodiments, the polyamine comprises primary and/or secondary amino groups.

In another preferred embodiment, in combination with any of the above or below embodiments, the first cationic polymer is a polyamine selected from polyallylamine, polyvinylamine, polybutylamine, polylysine, and copolymers thereof.

In a preferred embodiment, in combination with any of the above or below embodiments, the first cationic polymer is a polyvinylamine or a polyallylamine. The polyvinylamines and polyallylamines include linear or branched homopolymers of vinylamine or allylamine and copolymers of vinylamine or allylamine and amino- or amido-groups. In a further preferred embodiment, in combination with any of the above or below embodiments, the polyvinylamine is a linear or branched homopolymer of vinylamine or a copolymer of vinylamine and vinylformamide. Preferably, the copolymer of vinylamine and vinylformamide comprises 1% to 70% vinylformamide units, more preferably 2% to 40% vinylformamide units, most preferably 5% to 25% vinylformamide units, based on the total number of structural units of the polymer. In a further preferred embodiment, in combination with any of the above or below embodiments, the polyallyamine is a linear or branched homopolymer of allylamine.

In a preferred embodiment, in combination with any of the above or below embodiments, the first cationic polymer has a weight average molecular weight (Mw) of 1,000 to 500,000 Da, more preferably 1,000 to 100,000 Da, and most preferably 2,000 to 80,000 Da.

In the present specification, the weight average molecular weight (Mw) of a polymer is determined by size exclusion chromatography (SEC) coupled to multi-angle-light scattering and refractive index detectors (SEC-MALS-RI).

In the present specification, the term "hydrolysis degree" refers to the "hydrolysis degree of the formamide groups of the polymer".

In a further preferred embodiment, in combination with any of the above or below embodiments, the first cationic polymer is a polyvinylamine or polyallylamine having a weight average molecular weight (Mw) of 10,000 to 100,000 Da, preferably 20,000 to 80,000 Da, more preferably 25,000 to 50,000 Da and a hydrolysis degree of the formamide groups of 66% to 99%, preferably 67% to 90%, even more preferably 68% to 80%, and most preferably 68% to 75%.

In the present specification, the hydrolysis degree of the formamide groups of the polymer is determined by $^1$H-NMR according to the following method:

5.25 g of the polymer is weighted into a flask and 10 ml of water is added. The obtained mixture is rotated to get a homogenous composition and finally evaporated at 50°C under vacuum until a dry solid is observed. The solid is dried under high vacuum ($\leq$0.1 mbar) in an oven at 80°C for 15 h to yield a dry residue.

The degree of hydrolysis is determined by $^1$H-NMR (400 MHz apparatus from Brucker, solvent: $D_2O$) based on the quantification of hydrolysed groups versus total hydrolysable groups according to the method described in reference:

Q. Wen, A. M. Vincelli, R. Pelton, "Cationic polyvinylamine binding to anionic microgels yields kinetically controlled structures", J Colloid Interface Sci. 369 (2012) 223-230.

In a further preferred embodiment, in combination with any of the above or below embodiments, the first cationic polymer is cross-linked to a cross-linking degree of 4 to 25%. In a preferred embodiment, in combination with any of the above or below embodiments, the cross-linking degree is 5 to 20%, preferably 7 to 18%, more preferably 8 to 16%.

In the present specification, the "cross-linking degree" is defined as the cross-linker/polymer ratio (also referred to as "cross-linker ratio"). The "cross-linker ratio" is defined as the percentage in mol of the cross-linker versus the vinylamine structural units present in the polymer solution (based on average molecular weight) used for the reaction.

That is, the cross-linker ratio is calculated by the following formula (1):

$$(1) \quad \text{cross linker ratio} = \frac{V1 \times d1 \times C1 \times Mw2}{W2 \times C2 \times Mw1} \times 100\%$$

wherein V1 (ml) is the volume of cross-linker, d1 (g/ml) is the density of cross-linker, C1 (wt%) is the concentration of cross-linker, W2 (g) is the weight of polymer solution, C2 (wt%) is the concentration of polymer, Mw1 (g/mol) is the molecular weight of cross-linker and Mw2 (g/mol) is the average monomer unit molecular weight.

Mw2 is calculated by the following formula (2):

$$(2) \quad Mw2 = \left(\sum_k Nk \times Mk\right) / \sum_k Nk$$

wherein Nk is the number of monomer units of type k forming the polymer and Mk is the molecular weight (g/mol) of a monomer unit of type k.

The second anionic polymer preferably contains at least one functional group selected from carboxyl (-COOH).

In a preferred embodiment, in combination with any of the above or below embodiments, the second anionic polymer is selected from polyacrylic acid, poly(meth)acrylic acid, polyacrylamide-co-acrylic acid and partly cross-linked polymers thereof.

In a preferred embodiment, in combination with any of the above or below embodiments, the second anionic polymer has a weight average molecular weight (Mw) of at least 10,000 Da, preferably 20,000 to 2,000,000 Da, more preferably 25,000 to 500,000 Da, further preferably 30,000 to 300,000, even more preferably 50,000 to 300,000 Da, most preferably 80,000 to 250,000 Da.

In another preferred embodiment, in combination with any of the above or below embodiments, the weight average molecular weight (Mw) of the second anionic polymer is higher than the weight average molecular weight (Mw) of the first cationic polymer.

In a further preferred embodiment, in combination with any of the above or below embodiments, the first cationic polymer has a weight average molecular weight (Mw) of 10,000 to 100,000, preferably 15,000 to 50,000, more preferably 20,000 to 30,000, and the second anionic polymer has a weight average molecular weight (Mw) of 25,000 to 500,000, preferably 150,000 to 300,000, more preferably 200,000 to 250,000.

In a preferred embodiment, in combination with any of the above or below embodiments, the total concentration of first cationic polymer and second anionic polymer is at least 3% w/w, preferably at least 5% w/w, more preferably, at least 7% w/w, and is preferably less than 25% w/w, more preferably less than 20% w/w, most preferably less than 15%, based on the total weight of the dry composite material.

The composite material of the present invention may comprise additional layers of polymers, which can be cross-linked, partially cross-linked, or non-crosslinked polymers, said non-crosslinked polymers being covalently bonded to the cross-linked polymer-filled porous support and/or to the second non-crosslinked polymer.

In a preferred embodiment, in combination with any of the above or below embodiments, the composite material comprises 1 to 3 additional layers of non-crosslinked polymers, which can be the same or different than the second polymer. Said additional layers of non-crosslinked polymers should be covalently bonded to each other and to the cross-linked polymer-filled porous support and/or to the second polymer.

In another preferred embodiment, in combination with any of the above or below embodiments, the composite material does not comprise additional layers of non-crosslinked polymers (i.e. the only polymers present in the composite material are the first polymer and the second polymer).

METHOD FOR PRODUCING THE COMPOSITE MATERIAL

The composite material of the present invention can be produced according to the following method:

a) soaking a porous support with a solution or a dispersion containing a first cationic polymer, a cross-linker, and a solvent;

b) cross-linking the first cationic polymer with the cross-linker at a temperature below 250°C, and

c) covalently bonding a second anionic polymer to the external surface of the composite material obtained in step b).

Any cross-linker having at least two reactive groups can be used in the present invention.

In a preferred embodiment, in combination with any of the above or below embodiments, the cross-linker is selected from bis-epoxides, dialdehydes, and diglycidylethers. In a more preferred embodiment, in combination with any of the above or below embodiments, the cross-linker is selected from propanediol diglycidylether, butanediol diglycidylether, hexanediol diglycidylether, polyethylene glycol diglycidyl ether, glutaric dialdehyde and succinic dialdehyde. More preferably, the cross-linker is selected from butanediol diglycidylether and hexanediol diglycidylether.

If the first cationic polymer does not comprise cross-linkable groups, prior to step a) of the above method, the first cationic polymer is functionalized with cross-linkable groups by any method known in the art.

In a preferred embodiment, in combination with any of the above or below embodiments, the cross-linker ratio is 5 to 20% (mol/mol), more preferably 7 to 18% (mol/mol), and most preferably 8 to 16% (mol/mol).

Any solvent or medium capable of dissolving or dispersing the polymer and the cross-linker may be used provided that it does not react or only slowly reacts with the cross-linker and the polymer under the conditions of step b) of the above method. Slowly, in this context, means that no observable reaction between the cross-linker and the solvent and between the polymer and the solvent occurs for the duration of step b).

In a preferred embodiment, in combination with any of the above or below embodiments, the solvent is a polar protic or a polar aprotic solvent. In a preferred embodiment, in combination with any of the above or below embodiments, the solvent is a polar protic solvent selected from water, $C_{1-6}$ alcohols (e.g. methanol, ethanol, isopropanol, and butanol) and mixtures thereof. Water is most preferred.

In a preferred embodiment, in combination with any of the above or below embodiments, the pH of the polymer-cross-linker solution employed in step a) is adjusted to 8 to 13, preferably 9 to 11, most preferably 10 to 11. The pH adjustment can be carried out by adding a strong base such as NaOH or KOH.

During step b) of the above method, the temperature is preferably between 20 to 180°C, more preferably 40 to 100°C, and most preferably 50°C and 80°C.

In a preferred embodiment, in combination with any of the above or below embodiments, the duration of step b) is preferably between 1 hour and 100 hours, more preferably between 8 to 60 hours, and most preferably between 18 hours and 48 hours.

In a further preferred embodiment, in combination with any of the above or below embodiments, step b) is carried out at 40 to 100°C for 8 to 60 hours, preferably at 50 to 80°C for 12 to 50 hours, more preferably at 60°C for 24 to 48 hours.

In the present specification, the terms "covalently bonding" and "immobilization" are used interchangeably.

The immobilization of the second anionic polymer to the external surface of the composite material obtained in step b) can be achieved by any means known in the art. Preferably, the immobilization is obtained by an amide forming reaction between the polycarboxylic acid and the polyamine, such as amide bonding via heating, in the presence of a coupling agent and/or by anhydride activation of the carboxyl group.

In a preferred embodiment, in combination with any of the above or below embodiments, the immobilization of the second anionic polymer is carried out in the presence of an amine and carboxyl group coupling agent. More preferably, the coupling agent is a carbodiimide, particularly preferably 1-ethyl-3-(3-(dimethylamino)propyl)-N-carbodiimide (EDC, N-(3-(dimethylamino)propyl)-N-ethylcarbodiimide) or N',N'-dicyclohexyl carbodiimide (DCC).

In a further preferred embodiment, in combination with any of the above or below embodiments, N-hydroxysuccinimide (NHS) or its water-soluble analog (Sulfo-NHS) is included when EDC is used as coupling agent to improve efficiency.

In a further preferred embodiment, in combination with any of the above or below embodiments, the second anionic polymer is dissolved in a suitable solvent or medium. Any solvent capable of dissolving the second anionic polymer may be used provided that it does not react or only slowly reacts with the polymer under the conditions of step c) of the above method. Slowly, in this context, means that no observable reaction between the second polymer and the solvent occurs for the duration of step c).

In a preferred embodiment, in combination with any of the above or below embodiments, the solvent for the second anionic polymer is a polar protic or a polar aprotic solvent. In a preferred embodiment, in combination with any of the above or below embodiments, the solvent for the second anionic polymer is a polar protic solvent selected from water, $C_{1-6}$ alcohols (e.g. methanol, ethanol, isopropanol, and butanol) and mixtures thereof. Water is most preferred.

In the present specification, the term "non-crosslinked polymer" refers to a polymer which has not been subjected to active crosslinking through the addition of a crosslinker to the polymer. Thus, for the embodiment in which the second anionic polymer is dissolved in a solvent, said solvent does not contain any cross-linkers.

In a further preferred embodiment, in combination with any of the above or below embodiments, the method further comprises a step d) of hydrolysing any unreacted cross-linkable groups of the cross-linker after step c).

## USES OF THE COMPOSITE MATERIAL

In the present specification, the terms "feedstock" and "feed" are used interchangeably.

In the present specification, the terms "biological material" and "biomolecules" are used interchangeably and include viral particles, virus-like particles, extracellular vesicles, cells or phages.

Virus-like particles are virus-derived structures made up of one or more different molecules with the ability to self-assemble, mimicking the form and size of a virus particle but lacking the genetic material so they are not capable of infecting the host cell.

In the present specification, the term "proteins" includes polypeptides. Such polypeptides preferably contain at least 20 amino acid residues, more preferably between 40 and 80 amino acid residues.

The composite material of the present invention is useful for purifying a target biological material in a feedstock.

In a preferred embodiment, in combination with any of the above or below embodiments, the feedstock comprises host cell proteins (HCPs), and DNA, and optionally RNA and other nucleic acids.

In the present invention, the feedstock optionally contains albumins, endotoxins, detergents and microorganisms, or fragments thereof.

The invention also provides a method for purifying a target biological material in a feedstock, said method comprising the steps of:

i) contacting the feedstock with a composite material according to the invention for a sufficient time;

ii) separating the composite material from the purified feedstock;

iii) optionally, isolating the purified biological material from the feedstock; and

iv) optionally, washing the composite material with a solvent and collecting the obtained solution for further processing.

In a preferred embodiment, in combination with any of the above or below embodiments, the target biological material is a viral particle, virus-like particle, extracellular vesicle, cell or phage.

In a preferred embodiment, in combination with any of the above or below embodiments, the solvent of the feedstock is water optionally containing buffer(s), salt(s) and/or modifier(s).

In a preferred embodiment, in combination with any of the above or below embodiments, the feedstock is a fermentation broth supernatant (before or after filtration) or a cell culture supernatant (CCS) comprising the target biological material and DNA, RNA, or other nucleic acids, and Host cell proteins (HCPs) as impurities.

In a preferred embodiment, in combination with any of the above or below embodiments, the composite material is used in a batch adsorption process. In this embodiment, in step i) of the purification method of the invention, the composite material is dispersed in the feedstock and in step ii), the composite material is separated from the feedstock (e.g. by centrifugation).

In another preferred embodiment, in combination with any of the above or below embodiments, the composite material is packed in a chromatography column.

In the method for recovering a target biological material of the present invention, the feedstock is contacted with the composite material according to the invention for a sufficient time. In a preferred embodiment, in combination with any of the below embodiments, the contact time is 1 min to 10 hours, preferably 3 min to 5 hours, more preferably 5 min to 1 hour.

In a preferred embodiment, in combination with any of the above or below embodiments, prior to contacting the composite material with the feedstock, the composite material is equilibrated in an aqueous solution with a pH below 8, preferably 3 to 7.5, more preferably 4 to 7, and most preferably 5 to 6. The pH of the aqueous solution can be adjusted with any suitable buffer. For example, monobasic acids or salts thereof can be used for adjusting the pH. Preferred monobasic acids are formic, acetic, sulfamic, hydrochloric, perchloric acid, and glycine. Preferred salts of the monobasic acids are ammonium, alkyl ammonium, sodium and potassium salts.

In a preferred embodiment, in combination with any of the above or below embodiments, the pH is adjusted with ammonium acetate.

In a further preferred embodiment, in combination with any of the above or below embodiments, the pH is adjusted with phosphate-buffered saline (PBS).

In a preferred embodiment, in combination with any of the above or below embodiments, the ratio of feedstock to composite material (volume of feed to weight of dry composite material) is in the range of 2:1 to 100:1, preferably 5:1 to 80:1, more preferably 10:1 to 70:1, most preferably 20:1 to 50:1. High ratios of feedstock to composite material are preferred from the viewpoint of achieving efficient utilization of the composite material.

In a preferred embodiment, in combination with any of the above or below embodiments, the composite material separated in step ii) of the above method, which contains adsorbed impurities, is subjected to an elution procedure to elute said impurities, thereby regenerating the composite material for further use.

The method for purifying a target biological material of the present invention may contain additional purification steps known in the art. Examples of such purification steps include ion exchange chromatography, addition of flocculation or precipitation agents, centrifugation, crystallization, affinity chromatography (e.g. employing separation media harboring Protein A, Protein G, or a combination thereof), membrane filtration, depth filtration (with diatomaceous earth or activated carbon) and application of a monolithic separation agent.

In a preferred embodiment, in combination with any of the above or below embodiments, steps i) and ii) of the method for separating a target protein of the invention are repeated in sequence multiple times (e.g. 2, 3, 4, 5, 6 times) using the same or different composite materials according to the present invention.

The following examples illustrate the invention.

## EXAMPLES

### A) Method for producing the composite material

The composite material of the present invention can be produced according to the following method:

a) soaking a porous support with a solution or a dispersion containing a first polymer, a cross-linker, and a solvent;

b) cross-linking the first polymer with the cross-linker at a temperature below 250°C; and

c) covalently bonding a second polymer to the external surface of the composite material obtained in step b).

Materials

a. Silica gel

| No | Compound | Particle size | Pore size | Supplier |
|----|----------|---------------|-----------|----------|
| 1 | Porous silica gel | 35 $\mu$m | 35 nm | AGC Si Tech |
| 2 | Porous silica gel | 50 $\mu$m | 50 nm | AGC Si Tech |

b. First cationic polymer

| | Compound | Molecular weight | Supplier |
|---|----------|------------------|----------|
| A | Copolymer of 2-propen-1-amine with N-2-propenyl-2-propen-1-amine (polyallylamine-diallylamine copolymer) | Mw 20,000 | Nittobo |

c. Second anionic polymer

| | Compound | Molecular weight | Supplier |
|---|----------|------------------|----------|
| B | Poly(acrylic acid) | Mw ~50, 000 | Polyscience |
| C | Poly(acrylic acid) | Mv ~450, 000 | SIGMA |

d. Starting materials for composite materials

|  | Silica | First polymer | Cross-linker for first polymer | Second layer | Coupling reagent for second polymer |
|---|---|---|---|---|---|
| Comparative Example 1 | 1 | A | BDGE | None | None |
| Example 1 | 1 | A | BDGE | B | EDC |
| Example 2 | 1 | A | BDGE | C | EDC |
| Example 3 | 2 | A | BDGE | C | EDC |
| BDGE: 1,4-butanediol diglycidyl ether<br>EDC: N-(3-dimethylaminopropyl)-N-ethylcarbodiimide hydrochloride | | | | | |

Comparative Example 1

4.2 g of aqueous solution of polymer A (40% of polymer A in the solution) was added in 20 g of water and the pH of the solution was adjusted at 9.5 by 2M aqueous NaOH solution. 500 $\mu$L of 1,4-butanediol diglycidyl ether was mixed with the solution.

15 g of silica gel 1, dry powder, were sedimented into a flat bottom stainless steel dish. 26.0 g of the polymer-cross-linker solution were added dropwise and equally distributed over the porous support and mixed using a spatula. The resultant paste was shaken for 1 min on a gyratory shaker at 600 rpm, in order to obtain a homogeneous mass with smooth surface. After covering the dish with a stainless-steel lid, the paste was heated without further mixing or moving for 16 hours in an oven at 60°C yielding a moist composite.

45 mL of water was added in the dish and the slurry was filtrated. Subsequently, the wet cake was washed on a frit with 3 x 45 ml of water. Then, the composite cake was suspended into 90 ml of 0.2 N hydrochloric acid in an Erlenmeyer flask and shaken for 20 min at 40°C. Finally, the cake was washed on a frit with 6 x 25 ml of water.

Example 1

Step1: Coating and cross-linking reaction

4.2 g of aqueous solution of polymer A (40% of polymer A in the solution) was added in 20 g of water and the pH of the solution was adjusted at 9.5 by 2M aqueous NaOH solution. 500 $\mu$L of 1,4-butanediol diglycidyl ether was mixed with the solution.

15 g of silica gel 1, dry powder, were sedimented into a flat bottom stainless steel dish. 26.0 g of the polymer-cross-linker solution were added dropwise and equally distributed over the porous support and mixed using a spatula. The resultant paste was shaken for 1 min on a gyratory shaker at 600 rpm, in order to obtain a homogeneous mass with smooth surface. After covering the dish with a stainless-steel lid, the paste was heated without further mixing or moving for 16 hours in an oven at 60°C yielding a moist composite.

45 mL of water was added in the dish and the slurry was filtrated. Subsequently, the wet cake was washed on a frit with 3 x 45 ml of water. Then, the composite cake was suspended into 90 ml of 0.2 N hydrochloric acid in an Erlenmeyer flask and shaken for 20 min at 40°C. Finally, the cake was washed on a frit with 6 x 25 ml of water.

Step 2: Immobilization of second polymer

An aliquot of 5 g of the moist composite prepared in step 1 was suspended into 10 mL of water with 0.126 g of 25% polymer B aqueous solution in an Erlenmeyer flask and shaken for 10min at room temperature. 191 $\mu$L of N,N-diisopropylethylamine and 0.10 g of N,N-dimethyl-4-aminopyridine were added into the flask and shaken for 1 minutes. Subsequently, 0.20 g of N-(3-dimethylaminopropyl)-N-ethylcarbodiimide hydrochloride (EDC) was added into the flask and it was shaken for 4 hours at 25°C.

The slurry was filtrated and washed on a frit with 2 x 15 ml of water. Then, the cake was suspended in 10 mL of 0.2 N hydrochloric acid in an Erlenmeyer flask and shaken for 20 min. Finally, the cake was washed on a frit with 6 x 15 ml of water and then stored in 20% ethanol-water.

Example 2

Immobilization of second polymer

An aliquot of 10 g of the moist composite prepared in step 1 of example 1 was suspended into 20 mL of water with 63 mg of polymer C aqueous solution in an Erlenmeyer flask and shaken for 10min at room temperature. 191 μL of N,N-diisopropylethylamine and 0.10 g of N,N-dimethyl-4-aminopyridine were added into the flask and shaken for 1 minutes. Subsequently, 0.20 g of N-(3-dimethylaminopropyl)-N-ethylcarbodiimide hydrochloride was added into the flask and it was shaken for 4 hours at 25°C.

The slurry was filtrated and washed on a frit with 2 x 15 ml of water. Then, the cake was suspended in 10 mL of 0.2 N hydrochloric acid in an Erlenmeyer flask and shaken for 20 min. Finally, the cake was washed on a frit with 6 x 15 ml of water and then stored in 20% ethanol-water.

Example 3

Step1: Coating and cross-linking reaction

6.5 g of aqueous solution of polymer A (40% of polymer A in the solution) was added in 31 g of water and the pH of the solution was adjusted at 9.5 by 2M aqueous NaOH solution. 754 μL of 1,4-butanediol diglycidyl ether was mixed with the solution.

15 g of silica gel 2, dry powder, were sedimented into a flat bottom stainless steel dish. 33 g of the polymer-cross-linker solution were added dropwise and equally distributed over the porous support and mixed using a spatula. The resultant paste was shaken for 1 min on a gyratory shaker at 600 rpm, in order to obtain a homogeneous mass with smooth surface. After covering the dish with a stainless-steel lid, the paste was heated without further mixing or moving for 16 hours in an oven at 60°C yielding a moist composite.

45 mL of water was added in the dish and the slurry was filtrated. Subsequently, the wet cake was washed on a frit with 3 x 45 ml of water. Then, the composite cake was suspended into 90 ml of 0.2 N hydrochloric acid in an Erlenmeyer flask and shaken for 20 min at 40°C. Finally, the cake was washed on a frit with 6 x 25 ml of water.

Immobilization of second polymer

An aliquot of 10 g of the moist composite prepared in step 1 of example 3 was suspended into 20 mL of water with 65 mg of polymer C aqueous solution in an Erlenmeyer flask and shaken for 10min at room temperature. 196 μL of N,N-diisopropylethylamine and 0.11 g of N,N-dimethyl-4-aminopyridine were added into the flask and shaken for 1 minute. Subsequently, 0.20 g of N-(3-dimethylaminopropyl)-N-ethylcarbodiimide hydrochloride was added into the flask and it was shaken for 4 hours at 25°C.

The slurry was filtrated and washed on a frit with 2 x 15 ml of water. Then, the cake was suspended in 10 mL of 0.2 N hydrochloric acid in an Erlenmeyer flask and shaken for 20 min. Finally, the cake was washed on a frit with 6 x 15 ml of water and then stored in 20% ethanol-water.

**B) Application**

A method for purifying a target biological material in a feedstock. Particularly to separate a virus, viral like particle and/or extracellular vesicle of interest from contaminants (HcDNA, HCP, additive proteins) in a cell culture medium.

Protocol of Batch purification of Adeno associated virus (AAV):

- A slurry of the target material in 20% ethanol-water is prepared and transferred to a filtration device or beaker;

- Exchange the storage buffer to 3-5 column volume (CV) of 50 mM TrisHCl buffer (pH 7.2) by repeated filtration for at least 3 times to obtain a moist composite;

- Add the same volume of 50 mM Tris-HCl buffer to the moist composite to obtain a 50% slurry of beads in 50 mM TrisHCl buffer (pH 7.2);

- Add 50% slurry of beads into a 0.5 mL of centrifugal filter tube and spin to remove the buffer;

- Add feed into the centrifugal filter tube;

- Agitate the mixture in the tube by a suitable shaker for 30 minutes at room temprature;

- Centrifuge the tube with a clean collector tube under 10,000 x g for 1 min;

- Analyze the flow through fraction.

Adeno associated virus (AAV) purification:

Characteristic of feed: Supernatant of Cell line HEK293 CCS containing AAV

| CCS No. | AAV serotype | AAV (capsids/mL) | DNA (ng/$\mu$L) | HCP (ng/mL) |
|---------|--------------|------------------|-----------------|-------------|
| 1 | AAV8 | $1.0 \times 10^{12}$ | 10.32 | 20494 |
| 2 | AAV9 | $4.9 \times 10^{12}$ | 2.23 | 2581 |

Depletion performance and AAV recovery of the composites

| Composite material (Resin) | volume of resin | Feed | Feed volume | Recovery HCP (%) | Recovery DNA (%) | Recovery AAV (%) |
|---|---|---|---|---|---|---|
| Example 1 | 20 $\mu$L | CCS-1 | 200 $\mu$L | 30 | <1 | 76 |
| Comparative example 1 | 20 $\mu$L | CCS-1 | 200 $\mu$L | 19 | 1 | 6 |
| Commercial Benchmark-1* CaptoCore400 | 20 $\mu$L | CCS-1 | 200 $\mu$L | 22 | 34 | 88 |
| Example 2 | 20 $\mu$L | CCS-2 | 200 $\mu$L | 3 | 1 | 78 |
| Comparative example 1 | 20 $\mu$L | CCS-2 | 200 $\mu$L | 3 | 1 | 56 |
| Commercial Benchmark-1* CaptoCore400 | 20 $\mu$L | CCS-2 | 200 $\mu$L | 3 | 23 | 68 |
| Commercial Benchmark-2* CaptoCore700 | 20 $\mu$L | CCS-2 | 200 $\mu$L | 2 | 9 | 14 |
| * Capto Core 400 multimodal chromatography resin (supplier: Cytiva (cytivalifesciences.com))<br>* Capto Core 700 multimodal chromatography resin (supplier: Cytiva (cytivalifesciences.com)) | | | | | | |

Analytical method:

| AAV8 recovery determination | AAV8 Titration ELISA | PROGEN |
|---|---|---|

(continued)

| AAV9 recovery determination | AAV9 Titration ELISA | PROGEN |
|---|---|---|
| HCP recovery determination | HEK 293 HCP ELISA Kit, 3G | CYGNUS |
| DNA recovery determination | Quant-iT™ dsDNA Assay Kits, high sensitivity (HS) and broad range (BR) | Invitrogen (Thermo Fisher SCIENTIFIC) |

AAV determination:

PROGEN AAV Titration ELISA Kits were used to quantify the CCSs. The quantitative of AAV8 and AAV9 in CCSs were used the AAV-8 Titration ELISA Kit (#PRAAV8) and the AAV-9 Titration ELISA Kit (#PRAAV9) respectively, from PROGEN, Heidelberg (Germany) according to the manufacturer's instructions (manual "PRAAV8 ELISA en_V11", "PRAAV9 ELISA en_V02"), on a Infinite M Nano plus microplate reader and corresponding software from Tecan (Switzerland) for reading and data evaluation.

AAV Recovery (%) =100 x (AAV concentration in CCS) / (AAV concentration in the filtered supernatant).

Host Cell Protein (HCP) determination:

Cygnus HEK293 HCP Elisa Kit 3G was used to determine the efficiency of depletion of host cell proteins (HCPs), HEK293T Host Cell Proteins 3<rd>Generation (#F650S), from Cygnus Technologies, South port (USA) according to the manufacturer's instructions (manual"800-F650S, Rev. 01, 06JUL2021"), on a Infinite M Nano plus microplate reader and corresponding software from Tecan (Switzerland) for reading and data evaluation. Samples were diluted in the sample diluent (Product catalog number #I700 purchased from Cygnus Technologies).
The HCP Recovery is expressed as:

HCP Recovery (%) =100 x (HCP concentration in CCS) / (HCP concentration in the filtered supernatant)

DNA determination:

The samples to be analyzed were the CCSs.
The DNA quantification was accomplished utilizing DNA-specific fluorescence assay using Quant-iT™ dsDNA Assay Kits, high sensitivity (HS) and broad range (BR) (#Q33120), Invitrogen (Germany), according to the manufacturer's instructions, on a on a Infinite M Nano plus microplate reader and corresponding software from Tecan (Switzerland) for reading and data evaluation.
The DNA Recovery is expressed as:

DNA Recovery (%) =100 x (DNA concentration in CCS) / (HCP concentration in the filtered supernatant).

Exosome purification:

Adipose tissue-derived mesenchymal stem cell culture supernatant containing exosomes was used in the following expreiments.

Protocol of Batch purification of exosomes:

[0144]   - A slurry of the target material in 20% ethanol-water is prepared and transferred to a filtration device or beaker;
- Exchange the storage buffer to 5 CV of physiological saline solution by repeated filtration for at least 3 times to obtain a moist composite;
- Add the same volume of physiological saline solution to the moist composite to obtain a 50% slurry of beads with physiological saline solution;
- Add 50% slurry of beads into a 0.5 mL of centrifugal filter tube and spin to remove the solution;
- Add feed into the centrifugal filter tube;
- Agitate the mixture in the tube by a suitable shaker for 10 minutes at room temprature;
- Centrifuge the tube with a clean collector tube under 10,000 x g for 1 min;
- Analyze the flow through fraction.

| Resin | Volume of resin | Feed volume | Recovery Exosome (%) | Residual Protein (%) |
|---|---|---|---|---|
| Example 3 | 20 $\mu$L | 200 $\mu$L | 75.9 | 1.5 |
| Commercial Benchmark-2 CaptoCore700 | 20 $\mu$L | 200 $\mu$L | 63.5 | 1.2 |

Analytical method:

| Exosome quantification | CD9/CD63 ELISA | Cosmo Bio |
|---|---|---|
| Protein quantification | Pierce 660nm Protein Assay | Thermo Fisher Scientific |

Exosome quantification:

CD9/CD63 ELISA Kit was used for the quantification of Exosome. All procesures were completely followed by the instruction manual. Quantification was performed using the attached CD9/CD63 fusion protein for standard curve and the recovery rate was calculated by the following formula.

$$\text{Exosome Recovery (\%)} = 100 \times (\text{Exosome concentration in CCS}) / (\text{Exosome concentration in the filtered supernatant}).$$

Protein quantifcation:

10 $\mu$L of each sample was mixed with 200 uL of protein assay reagent, and the absorbance at 660 nm wavelength was measured after 5 minutes. Bovine serum albumine (BSA) mixed in the same ratio was used for the standard curve. Measurements were perfomed using microplate reader. The residual protein was calculated by the following formula, the same as the recovery rate of exosomes.

$$\text{Residual Protein (\%)} = 100 \times (\text{Protein concentration in CCS}) / (\text{Protein concentration in the filtered supernatant})$$

**Claims**

1.  A composite material comprising:

     a porous support being filled with a first cationic polymer which is cross-linked, and

a second anionic polymer which is covalently bonded to the external surface of the cross-linked polymer-filled porous support.

2. The composite material according to claim 1, wherein the first cationic polymer contains primary amino functional groups

3. The composite material according to claim 2, wherein the first cationic polymer is selected from polyvinylamine, polyallylamine, polybutylamine, polylysine, or copolymers thereof.

4. The composite material according to any one of claims 1 to 3, wherein the first cationic polymer has a cross-linking degree of 4 to 25%.

5. The composite material according to any one of claims 1 to 4, wherein the second anionic polymer contains at least one carboxyl group.

6. The composite material according to claim 5, wherein the second anionic polymer is selected from polyacrylic acid, poly(meth)acrylic acid, poly(acrylamide-co-acrylic acid) and partly cross-linked polymers thereof.

7. The composite material according to any one of claims 1 to 6, wherein the first cationic polymer has a weight average molecular weight (Mw) of 1,000 to 100,000 Da and/or the second anionic polymer has a weight average molecular weight (Mw) of at least 10,000 Da.

8. The composite material according to claim 7, wherein the first cationic polymer has a weight average molecular weight (Mw) of 2,000 to 80,000 Da and/or the second anionic polymer has a weight average molecular weight (Mw) of 20,000 to 2,000,000 Da.

9. The composite material of any one of claims 1 to 8, wherein the porous support has an average pore size of 5 to 500 nm and/or the porous support is a particulate and monolithic material with an average particle size of 1 to 500 μm.

10. A method for producing the composite material according to any of claims 1 to 9 comprising the steps of:

    a) soaking a porous support with a solution or a dispersion containing a first cationic polymer, a cross-linker, and a solvent;
    b) cross-linking the first cationic polymer with the cross-linker at a temperature below 250°C, and
    c) covalently bonding a second anionic polymer to the external surface of the composite material obtained in step b).

11. The method of claim 10, wherein the cross-linker is selected from propanediol diglycidylether, butanediol diglycidylether, hexanediol diglycidylether, glutaric dialdehyde and succinic dialdehyde.

12. The method of claim 10 or 11, wherein in step c) the second anionic polymer is covalently bonded to the external surface of the composite material by an amine and carboxyl group coupling agent.

13. Use of the composite material according to any one of claims 1 to 9 for purifying a target biological material in a feedstock.

14. A method for purifying a target biological material in a feedstock, said method comprising the steps of:

    i) contacting the feedstock with a composite material according to any one of claims 1 to 9;
    ii) separating the composite material from the purified feedstock;
    iii) optionally, isolating the purified target biological material from the feedstock; and
    iv) optionally, washing the composite material with a solvent and collecting the obtained solution for further processing.

15. The method according to claim 14, wherein the target biological material is a viral particle, virus-like particle, extracellular vesicle, cell or phage.

Europäisches
Patentamt

European
Patent Office

Office européen
des brevets

# EUROPEAN SEARCH REPORT

Application Number

EP 22 30 6901

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/039070 A1 (GOTTSCHALL KLAUS [DE] ET AL) 11 February 2021 (2021-02-11) * paragraph [0044] – paragraph [0047] * * paragraph [0053] – paragraph [0054] * * paragraph [0115] * * paragraph [0130] – paragraph [0131] * * paragraph [0226] – paragraph [0228] * * paragraph [0494] * * examples 1, 1a * * claim 20 * | 1-15 | INV. B01J20/26 A61K48/00 B01D15/08 B01J20/32 |
| A,D | WO 2019/170634 A1 (CHIRAL TECH EUROPE SAS [FR]) 12 September 2019 (2019-09-12) * the whole document * | 1-15 | |
| A | US 2004/202783 A1 (BAUMANN HANNO [DE] ET AL) 14 October 2004 (2004-10-14) * the whole document * | 1-15 | |
| A | EP 0 230 247 A2 (KANEGAFUCHI CHEMICAL IND [JP]) 29 July 1987 (1987-07-29) * the whole document * | 1-15 | TECHNICAL FIELDS SEARCHED (IPC) B01J B01D A61K |

The present search report has been drawn up for all claims

| Place of search | Date of completion of the search | Examiner |
|---|---|---|
| The Hague | 12 June 2023 | Klemps, Christian |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 30 6901

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2023

| Patent document cited in search report | | Publication date | Patent family member(s) | | Publication date |
|---|---|---|---|---|---|
| US 2021039070 | A1 | 11-02-2021 | CN | 111836824 A | 27-10-2020 |
| | | | EP | 3765481 A2 | 20-01-2021 |
| | | | US | 2021039070 A1 | 11-02-2021 |
| | | | WO | 2019175412 A2 | 19-09-2019 |
| WO 2019170634 | A1 | 12-09-2019 | CN | 111818981 A | 23-10-2020 |
| | | | EP | 3762121 A1 | 13-01-2021 |
| | | | JP | 7196203 B2 | 26-12-2022 |
| | | | JP | 2021515698 A | 24-06-2021 |
| | | | JP | 2022184990 A | 13-12-2022 |
| | | | US | 2021106974 A1 | 15-04-2021 |
| | | | WO | 2019170634 A1 | 12-09-2019 |
| US 2004202783 | A1 | 14-10-2004 | AT | 332188 T | 15-07-2006 |
| | | | AU | 2159602 A | 26-03-2002 |
| | | | DE | 10045434 A1 | 04-04-2002 |
| | | | EP | 1326708 A2 | 16-07-2003 |
| | | | ES | 2267838 T3 | 16-03-2007 |
| | | | JP | 2004508188 A | 18-03-2004 |
| | | | US | 2004202783 A1 | 14-10-2004 |
| | | | WO | 0222253 A2 | 21-03-2002 |
| EP 0230247 | A2 | 29-07-1987 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019170634 A
- US 2016367966 A
- US 2019111419 A
- WO 2019206940 A
- WO 2019006390 A
- US 9782468 B
- WO 2021092193 A
- US 2021188903 A
- WO 95025574 A
- US 6783962 B
- WO 2004073843 A
- EP 2545989 A
- WO 2018050849 A
- WO 2006015495 A
- WO 2005120701 A
- EP 2027921 A
- WO 2011140406 A

**Non-patent literature cited in the description**

- **K. REITER et al.** Separation of virus-like particles and extracellular vesicles by flow-through and heparin affinity chromatography. *Journal of Chromatography A,* vol. 1588, 77-84
- **Q. WEN ; A. M. VINCELLI ; R. PELTON.** Cationic polyvinylamine binding to anionic microgels yields kinetically controlled structures. *J Colloid Interface Sci.,* 2012, vol. 369, 223-230